# EUROPEAN PATENT APPLICATION

(11) **EP 4 131 183 A1**
(43) Date of publication of application: **08.02.2023**
(21) Application number: 21203743.6
(22) Date of filing: 20.10.2021
(51) Int. Cl.: G06V 30/10, G16H 15/00, G16H 30/40

(54) **METHOD FOR EXTRACTING THERAPY AND USAGE DATA FROM A MEDICAL DEVICE**

(30) Priority: 05.08.2021 SG 10202108562X
(71) Applicant: Advanced MedTech Corporate Pte. Ltd., Singapore 608526 (SG)
(72) Inventor: Lim, Kenny Tse Yang, Singapore (SG); Devarajaiah Chikkanaravangala, Shadakshari, Singapore (SG)
(74) Representative: Grünecker Patent- und Rechtsanwälte PartG mbB

(57) **Abstract**

The invention refers to a data extraction system 1 comprising a medical device 3 with a display 4, and separate from the medical device, a data extraction unit 2. The data extraction unit 2 comprises an optical sensor 9 capable of taking a picture of at least a part of the display, and a processing unit 10 capable to convert image information comprised by the picture to data. The data comprises at least one of textual data and numerical data. Further, the invention refers to a method for extracting data from a display of a medical device using the data extraction unit. The method comprises taking a picture of at least a part of the display of the medical device by the optical sensor, and converting image information comprised by the picture to data by the processing unit.

## Description

The present invention refers to a data extraction system according to claim 1 and a method for extracting data from a display of a medical device according to claim 8.

### Prior art

The article "Real-Time Detection and Reading of LED/LCD Displays for Visually Impaired Persons" by E. Tekin et al. refers to a system that acquires video, detects and reads LED/LCD characters in real time, reading them aloud to a user with synthesised speech.

Most of the existing medical devices are not provided with an inbuilt capability for transmitting data out either by wired or wireless connectivity. Thus, it is very difficult to get both the therapy related data and the service data out of the device, e.g., for status check, trending, data analysis, therapy compliance monitoring, and/or preventive maintenance.

### Object

The invention thus has the object to provide a data extraction system and a method for extracting data from a display of a medical device that enables a non-invasive way of extracting data from an existing medical device using a simple mechanism.

### Solution

This object is solved by the data extraction system of claim 1 and the method for extracting data from a display of a medical device of claim 8. Additional embodiments and improvements are disclosed in the dependent claims.

The data extraction system comprises a medical device with a display, and separate from the medical device, a data extraction unit. The data extraction unit comprises an optical sensor capable of taking a picture of at least a part of the display, and a processing unit capable to convert image information comprised by the picture to data. The data comprises at least one of textual data and numerical data.

The data extraction unit allows in a comfortable way to extract data from image information comprised by a picture being taken from the display of the medical device.

The medical device, e.g., a medical device produced at former times, may be provided without an inbuilt capability for transmitting data out either by wired or wireless connectivity. Thus, the data extraction unit allows to get out at least one of textual data and numerical data, e.g., therapy related data and/or service data, e.g., for status check, trending, data analysis, therapy compliance monitoring, and/or preventive maintenance.

However, it is also possible that the medical device may be provided with an inbuilt capability for transmitting data out either by wired or wireless connectivity. In such a case, nevertheless, the data extraction unit may be used to allow to get out at least one of textual data and numerical data without using the inbuilt capability of the medical device for transmitting data out either by wired or wireless connectivity, This may be advantageous, e.g., when a user is not familiar with using the inbuilt capability of the medical device for transmitting data out either by wired or wireless connectivity, and/or e.g., as by using the data extraction unit - both for medical devices without and with an inbuilt capability for transmitting data out either by wired or wireless connectivity - a consistent experience for a user of different medical devices can be achieved.

The optical sensor can further be capable of taking several pictures of at least a part of the display.

The processing unit capable to convert image information comprised by each of the several pictures to respective data. Each one of the respective data may be assigned to one of the several image information of the several pictures.

The data can comprise at least one of therapy data and usage data from the medical device.

Therapy data may comprise information about measurements and/or treatments that are or are to be performed and/or settings that are or are to be used during one or more measurements and/or treatments. The usage data may comprise information about actually performed measurements and/or treatments and/or information about actually used settings during the one or more measurements and/or treatments.

The data extraction unit can further comprise a transmission unit for transmitting the data to a backend server for processing the data to processed data and storing the data and/or storing the processed data.

The data and/or the processed data may be stored at a storage of the backend server.

The data extraction unit can further comprise a retrieval unit for retrieving the data and/or the processed data from the backend server.

The retrieval unit may retrieve the data and/or the processed data from the storage of the backend server.

The data extraction unit can further comprise a display unit for displaying the retrieved data and/or the retrieved processed data. The display unit may also be capable of displaying the picture, e.g., alone or in combination with at least one of the retrieved data and the retrieved processed data. Exemplarily the display unit can be capable of displaying the retrieved data and/or the retrieved processed data in various modes.

By displaying the retrieved data and/or the retrieved processed data by the display unit of the data extraction unit no additional display device, being separate from the data extraction unit and separate from the medical device, is required.

Displaying the retrieved data and/or the retrieved processed data in various modes by the display unit may comprise status check, trending, data analysis, therapy compliance monitoring, and/or preventive maintenance.

The data and/or the retrieved data and/or the retrieved processed data can comprise at least one of:
- date, time, duration of a treatment,
- date of last treatment,
- diagrams,
- full name of a patient,
- treated disease,
- total number of applied shock waves,
- total energy of applied shock waves,
- different levels of the shock waves and their respective numbers,
- a list with columns with intensity level, number of shock waves per level, energy per level,
- a patients list comprising at least one of a list with names of various patients, a list with a last treatment overview naming the treated disease, a list with dates of the last visit, a list with the number of total sessions and a list with possible actions.

The data extraction unit can further comprise a report generating unit capable of generating a report comprising at least partly at least one of the data, the processed data, the retrieved data and the retrieved processed data, wherein exemplarily the report generating unit is further capable of sending the report to a remote device and/or the backend server.

The optical sensor and the processing unit can be comprised by a handheld device.

The transmission unit further can be comprised by the handheld device.

The retrieval unit further can be comprised by the handheld device.

The report generating unit further can be comprised by the handheld device.

The display unit further can be comprised by the handheld device.

Thus, the data extraction unit may be a handheld device.

The data extraction unit can be comprised by a support structure. Thus, the optical sensor and the processing unit, and, e.g., the transmission unit, the retrieval unit, the display unit and/or the report generating unit can be comprised by the support structure.

The support structure can comprise attaching means for attaching the support structure to the display of the medical device directly and/or for attaching the support structure to a tripod or the like.

The data extraction unit as described above or below can also be provided as a stand-alone device.

The method for extracting data from a display of a medical device using the data extraction unit as disclosed above or below comprises taking a picture of at least a part of the display of the medical device by the optical sensor, and converting image information comprised by the picture to data by the processing unit. The data comprises at least one of textual data and numerical data.

The method allows in a comfortable way extracting data from image information comprised by a picture being taken from the display of the medical device by using the data extraction unit.

The data can comprise at least one of therapy data and usage data from the medical device.

The data and the usage data can be used for proactive predictions using machine learning.

The method can further comprise transmitting, by the transmission unit, the data to a backend server for processing the data to processed data and storing the data and/or storing the processed data.

The method can further comprise retrieving, by the retrieval unit, the data and/or the processed data from the backend server.

The method can further comprise displaying, by the display unit, the retrieved data and/or the retrieved processed data.

The method can further comprise displaying, by the display unit, the retrieved data and/or the retrieved processed data in various modes.

The method can further comprise generating, by the report generating unit, a report comprising at least partly at least one of the data, the processed data, the retrieved data and the retrieved processed data.

The method can further comprise sending, by the report generating unit, the report to a remote device and/or the backend server.

The method can further comprise incorporating ultrasound and/or third party medical images to the report.

By incorporating the ultrasound and/or third party medical images to the report generated by the report generating unit, additional information, e.g., not being comprised by the picture of the display of the medical device may be added to the report.

The method can further comprise evaluating the data and/or the processed data to provide trending data. The trending data may quantify a development during a therapy and/or a development of the medical device.

### Brief description of the figures

The attached figures illustrate exemplified for better understanding and for visualisation aspects and embodiments of the invention.
Figure 1 illustrates schematically a data extraction system, a backend server and a remote device;
Figure 2 illustrates schematically the data extraction unit of the data extraction system;
Figure 3 illustrates an oblique view of taking a picture of the display of the medical device by the data extraction unit;
Figure 4 illustrates a front view of taking a picture of the display of the medical device by the data extraction unit;
Figure 5 illustrates the display unit displaying first data;
Figure 6 illustrates the display unit displaying second data and the taken picture; and
Figure 7 illustrates the display unit with a patients list.

### Detailed description of the figures

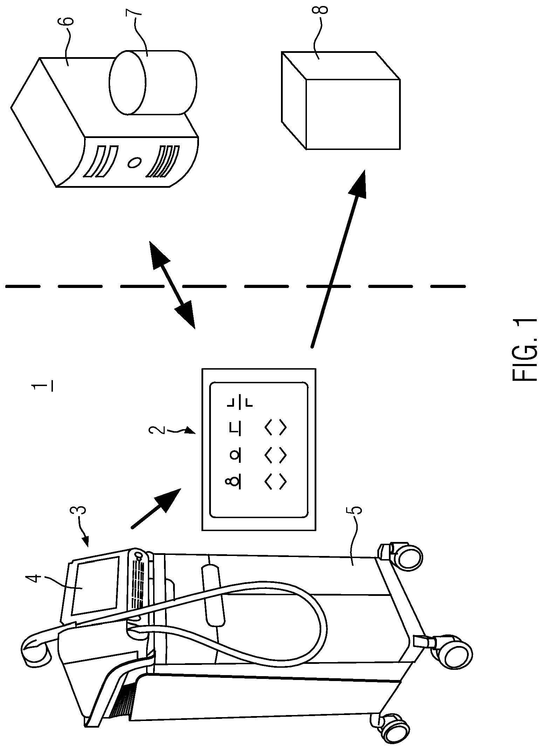

Figure 1 illustrates schematically a data extraction system 1, a backend server 6 and a remote device 8. The data extraction system 1 comprises a medical device 3 with a display 4 and a housing 5, and a data extraction unit 2. The data extraction unit 2 comprises a display unit 13. A picture of at least a part of the display 4 may be taken by the data extraction unit 2.

The data extraction unit 2 may convert image information comprised by the picture to data. The data comprises at least one of textual data and numerical data. The data extraction device 2 may transmit the data to the backend server for processing the data to processed data and storing the data and/or storing the processed data in the backend server 6 and/or a storage 7 of the backend server 6.

The data extraction unit 2 may retrieve the data and/or the processed data from the backend server and/or the storage 7.

The data extraction unit 2 may generate a report comprising at least partly at least one of the data, the processed data, the retrieved data and the retrieved processed data. The report may be send to a remote device and/or the backend server.

The data extraction unit 2 may display the retrieved data and/or the retrieved processed data and/or the captured image on the display unit 13.

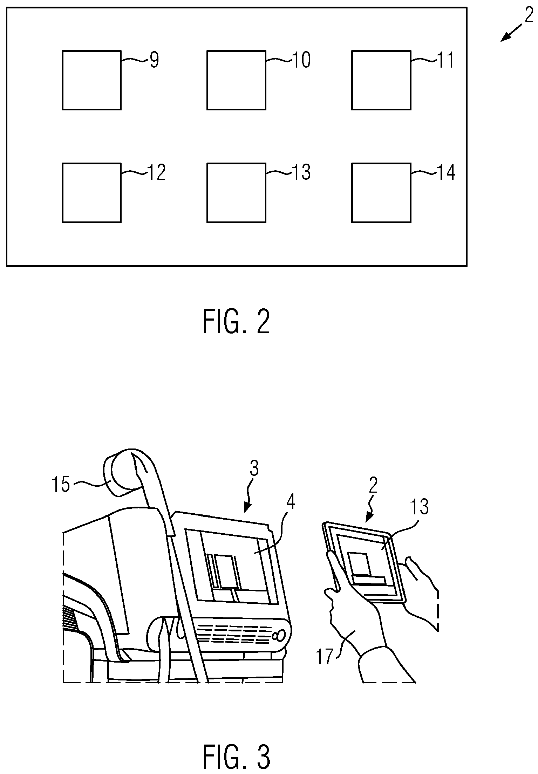

Figure 2 illustrates schematically the data extraction unit 2 of the data extraction system 1.

The data extraction unit 2 comprises an optical sensor 9 being capable of taking the picture of at least a part of the display 4. Further, the data extraction unit 2 comprises a processing unit 10 being capable to convert image information comprised by the picture to the data.

A transmission unit 11 of the data extraction device 2 is capable to transmit the data to the backend server for processing the data to the processed data and storing the data and/or storing the processed data in the backend server 6 and/or a storage 7 of the backend server 6.

A retrieval unit 12 of the data extraction unit 2 is capable to retrieve the data and/or the processed data from the backend server 6 and/or the storage 7.

A report generation unit 14 of the data extraction unit 2 is capable to generate the report comprising at least partly at least one of the data, the processed data, the retrieved data and the retrieved processed data. The report may be send to a remote device and/or the backend server by the report generating unit 14.

The data extraction unit 2 may display the retrieved data and/or the retrieved processed data and/or the captured image on the display unit 13.

Figure 3 illustrates an oblique view of taking a picture of the display 4 of the medical device 3 by the data extraction unit 2. In the depicted case, the medical device 3 comprises a head 15 for providing shockwaves. On the display unit 13 of the data extraction unit 2, the representation of the display 4 can be seen. The data extraction unit 2 is held by a user 17; however, alternatively, the data extraction unit 2 may be arranged on a tripod, or the like, set up at a distance from the display 4 of the medical device 3.

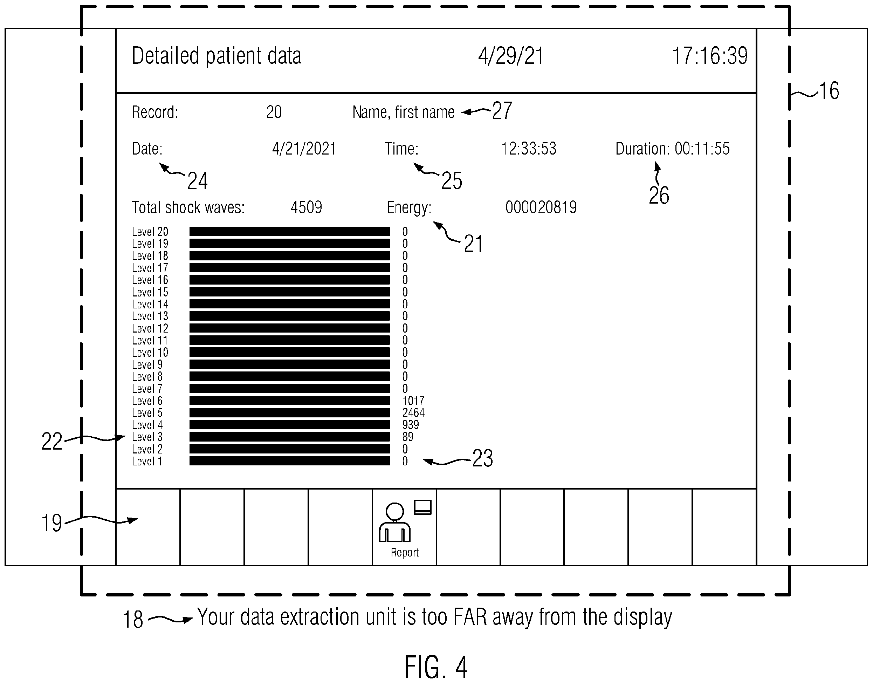

Figure 4 illustrates a front view of taking a picture of the display 4 of the medical device 3 by the data extraction unit 2. On the display unit 13 of the data extraction unit 2 the area of the picture to be taken is indicated by the surrounding frame 16. At a bottom region of the display unit 13, a notification 18 has popped up indicating "Your data extraction unit is too FAR away from the display".

On the display 4, an overview 19 of "Detailed patient data" is displayed. The overview 19 merely takes up about two-thirds of the area inside the surrounding frame 16. Therefore, the data extraction unit 2 may be moved nearer to the display 4 such that the overview 19 takes up, e.g., at least four-fifths of the area inside the surrounding frame 16.

The overview 19 displays information about a total number 20 of applied shock waves, the applied energy 21, different levels 22 of the shock waves and their respective numbers 23. Moreover, the date 24, the time 25 and the duration 26 of the treatment are displayed. The full name 27 of the patient may be displayed.

After taking a picture from the display 4 displaying the overview 19, image information comprised by the picture may be converted to data by processing unit 10 of the data extraction unit 2, wherein the data may comprise at least one of textual data and numerical data.

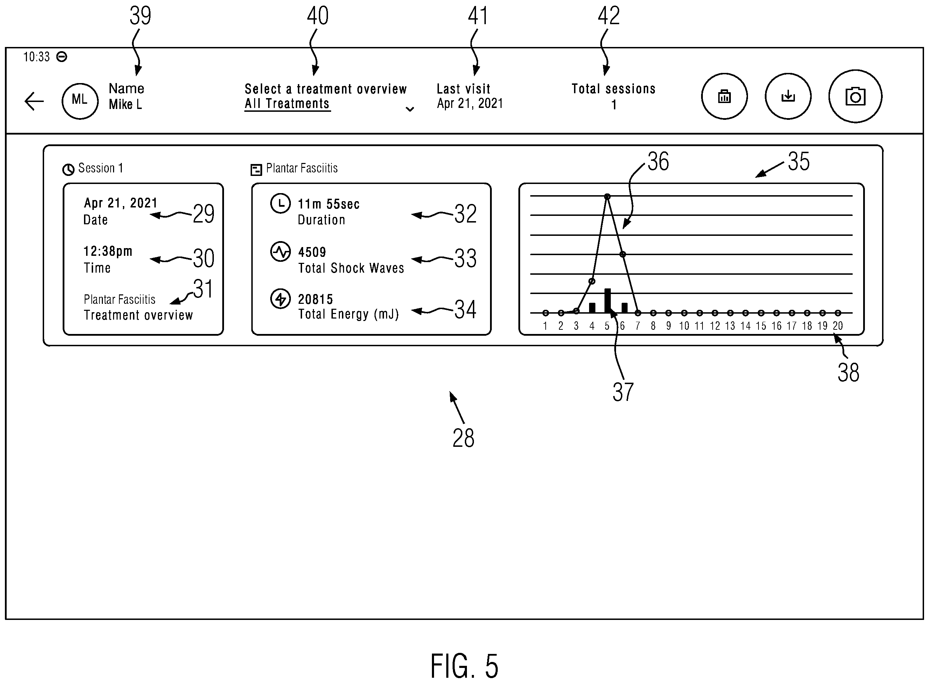

Figure 5 illustrates the display unit 13 displaying first data 28 of the data. The first data 28 comprises the date 29, the time 30 and the duration 32 of the treatment. The treated disease 31 is displayed as well as the total number 33 of shockwaves and the total energy 34 applied in the treatment. Moreover, the first data 28 comprises the name 39 of the patient, the selected overview 40, the date 41 of the last visit and the total number 42 of sessions.

In a diagram 35, the dependency of the number 37 of shock waves for the various levels 38 and the dependency of the energy 36 for the various levels are depicted.

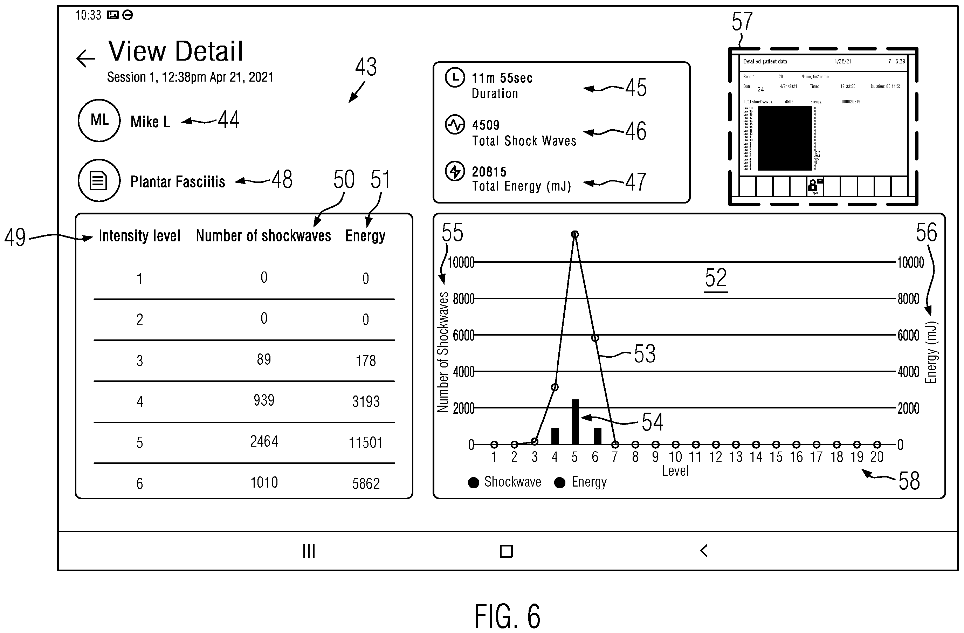

Figure 6 illustrates the display unit 13 displaying second data 43 and the taken picture 57. The second data 43 comprises the patient name 44, the duration 45 of the treatment, the total number 46 of shockwaves and the total energy 47 applied in the treatment. The treated disease 48 is also comprised by the second data 43. Moreover, a list with a column of intensity level 49, with a column of the number of shockwaves 50, and with a column of the energy 51 is comprised by the second data. In a diagram 52, the dependency of the number 54 of shock waves for the various levels 58 and the dependency of the energy 53 for the various levels 58 are depicted. The diagram comprises a first y-axis 55 relating to the number of shockwaves and a second y-axis 56 relating to the energy in mJ. Additionally the taken picture 57 from which the second data has been obtained is displayed.

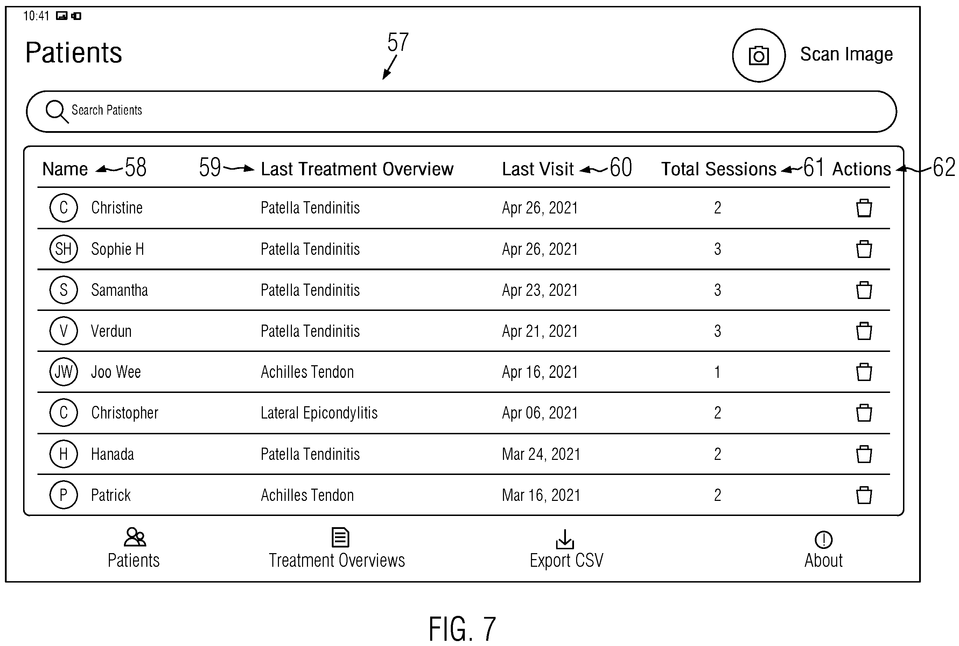

Figure 7 illustrates the display unit 13 with a patients list 57. The patients list 57 may comprise a list with names 58 of the various patients, a list with a last treatment overview 59 naming the treated disease, a list with dates of the last visit 60, a list with the number of total sessions 61 and a list with possible actions 62.

## Claims

1. A data extraction system (1) comprising:
a medical device (3) with a display (4);
separate from the medical device (3), a data extraction unit (2) comprising:
an optical sensor (9) capable of taking a picture of at least a part of the display (4);
a processing unit (10) capable to convert image information comprised by the picture to data, the data comprising at least one of textual data and numerical data.

2. The data extraction system of claim 1, the data comprising at least one of therapy data and usage data from the medical device (3).

3. The data extraction system of claim 1 or 2, the data extraction unit (2) further comprising a transmission unit (11) for transmitting the data to a backend server (6) for processing the data to processed data and storing the data and/or storing the processed data.

4. The data extraction system of claim 3, the data extraction unit (2) further comprising a retrieval unit (12) for retrieving the data and/or the processed data from the backend server (6).

5. The data extraction system of claim 4, the data extraction unit (2) further comprising a display unit (13) for displaying the retrieved data and/or the retrieved processed data and/or the picture, wherein exemplarily the display unit (13) is capable of displaying the retrieved data and/or the retrieved processed data in various modes.

6. The data extraction system of one of claims 1 to 5, the data extraction unit (2) further comprising a report generating unit (14) capable of generating a report comprising at least partly at least one of the data, the processed data, the retrieved data and the retrieved processed data, wherein exemplarily the report generating unit (14) is further capable of sending the report to a remote device (8) and/or the backend server (6).

7. The data extraction system of one of claims 1 to 6, wherein the optical sensor (9) and the processing unit (10) are comprised by a handheld device, wherein exemplarily the transmission unit (11) further is comprised by the handheld device, wherein exemplarily the retrieval unit (12) further is comprised by the handheld device, wherein exemplarily the display unit (13) further is comprised by the handheld device, wherein exemplarily the report generating unit (14) further is comprised by the handheld device.

8. A method for extracting data from a display (4) of a medical device (3) using the data extraction unit (2) of one of claims 1 to 7, the method comprising:
taking a picture of at least a part of the display (4) of the medical device (3) by the optical sensor (9);
converting image information comprised by the picture to data by the processing unit (10), the data comprising at least one of textual data and numerical data.

9. The method of claim 8, the data comprising at least one of therapy data and usage data from the medical device, exemplarily the method further comprising using the data and the usage data for proactive predictions using machine learning.

10. The method of claim 8 or 9, further comprising:
transmitting, by the transmission unit (11), the data to a backend server (6) for processing the data to processed data and storing the data and/or storing the processed data.

11. The method of claim 10, further comprising:
retrieving, by the retrieval unit (12), the data and/or the processed data from the backend server (6).

12. The method of claim 11, further comprising:
displaying, by the display unit (13), the retrieved data and/or the retrieved processed data, exemplarily displaying, by the display unit (13), the retrieved data and/or the retrieved processed data in various modes.

13. The method of one of claims 8 to 12, further comprising:
generating, by the report generating unit (14), a report comprising at least partly at least one of the data, the processed data, the retrieved data and the retrieved processed data, exemplarily sending, by the report generating unit (14), the report to a remote device (8) and/or the backend server (6).

14. The method of claim 13, further comprising:
incorporating ultrasound and/or third party medical images to the report.

15. The method of one of claims 8 to 14, further comprising:
evaluating the data and/or the processed data to provide trending data.
